# EUROPÄISCHE PATENTANMELDUNG

(11) **EP 3 025 590 A1**
(43) Veröffentlichungstag der Anmeldung: **01.06.2016**
(21) Anmeldenummer: 14194947.9
(22) Anmeldetag: 26.11.2014
(51) Int. Cl.: A23K 10/00, A23L 33/21, A23L 3/3454, A23L 3/3463, A23L 5/00, A23L 5/20, A61K 35/10

(54) **VERWENDUNG VON TORF**

(71) Anmelder: Weichselbaum, Johann, 3925 Arbesbach (AT)
(72) Erfinder: Weichselbaum, Johann, 3925 Arbesbach (AT)
(74) Vertreter: KLIMENT & HENHAPEL

(57) **Zusammenfassung**

Die vorliegende Erfindung betrifft die Verwendung von Torf zur Reduktion der Menge Phosphonate in einem phosphonathaltigen Ausgangsmaterial.

## Beschreibung

Die vorliegende Erfindung betrifft Mittel zur Reduktion der Menge von freien Phosphonaten in einem Ausgangsmaterial.

Phosphonate sind Salze und organische Verbindungen, die sich von der Phosphonsäure (H₃PO₃), auch bekannt als phosphorige Säure, ableiten lassen. Organische Phosphonat-Verbindungen unterscheiden sich von den Estern der Phosphorsäure durch die direkte Bindung des Phosphors an den Kohlenstoff eines Alkyl- oder Arylrests. Phosphonate können auch eine Aminogruppe aufweisen, wodurch diese in der Lage sind Metallionen zu komplexieren (vgl. mit Komplexbildern wie EDTA). Dadurch eignen sich bestimmte Gruppen von Phosphonaten u.a. besonders gut zum Enthärten von Wasser.

Phosphonate können aufgrund ihrer Eigenschaften auch als Herbizide eingesetzt werden. Wichtigster und bekanntester Vertreter dieser Gruppe von Herbiziden ist Glyphosat, welches seit mehr als 30 Jahren zur Bekämpfung von Unkraut eingesetzt wird. Glyphosat ist in der Lage das Enzym 5-Enolpyruvylshikimat-3-phosphat-Synthase zu blockieren, das zur Synthese der aromatischen Aminosäuren Phenylalanin, Tryptophan und Tyrosin über den Shikimisäureweg benötigt wird. Durch die Inhibierung dieses Enzyms ist die mit Glyphosat behandelte Pflanze nicht mehr in der Lage die zuvor genannten Aminosäuren zu synthetisieren und stirbt ab. Da der Shikimisäureweg ausschließlich bei Pflanzen und Mikroorganismen vorhanden ist, scheint Glyphosat ein auf den ersten Blick für Tiere und Menschen unbedenkliches Herbizid zu sein. Jedoch scheint es konkrete Hinweise zu geben, dass Glyphosat gegenüber Tieren genotoxisch, kanzerogen, zytotoxisch und teratogen sein könnte. Des Weiteren muss auch berücksichtigt werden, dass Glyphosat auch den Shikimisäureweg in Mikroorganismen inhibieren kann, so dass Glyphosat auch auf das Wachstum von Mikroorganismen Einfluss nehmen kann. Werden Mikroorganismen, die in Symbiose mit Tieren und/oder Menschen leben, inhibiert hat dies auch direkte Auswirkungen auf Tier und Mensch, insbesondere wenn sich die Zusammensetzung der üblicherweise vorkommenden Mikroorganismenflora ändert (z.B. im Magen-Darm-Trakt).

Nachdem es heute als gesichert gilt, dass Phosphonate wie Glyphosat über die behandelten Pflanzen in die Nahrungskette von Menschen und Tieren gelangt, ist es erstrebenswert - um die zuvor erwähnten Effekte von Phosphonaten zu vermeiden bzw. zu reduzieren - die Menge an Phosphonaten in Futtermitteln und Lebensmitteln zu reduzieren oder gänzlich zu entfernen.

Es ist eine Aufgabe der vorliegenden Erfindung Mittel und Verfahren bereitzustellen, um die Menge an Phosphonaten in einer Zusammensetzung zu reduzieren bzw. die Wirkung von Phosphonaten zu inhibieren.

Es hat sich erfindungsgemäß gezeigt, dass die Verwendung von Torf zu einer Reduktion der Menge von Phosphonaten, insbesondere freier Phosphonate, in einem phosphonathaltigen Ausgangsmaterial führt.

Torf ist ein organisches Sediment, welches aus nicht oder nur unvollständig zersetzten pflanzlichen Resten besteht. Torf entsteht aus abgestorbenen vorwiegend pflanzlichen Organismen unter Abschluss von Luftsauerstoff und in Anwesenheit von Wasser. Daher kann frisch gewonnener Torf bis zu 90% Wasser umfassen. Neben zum Teil unveränderten Pflanzenbestandteilen wie Zellulose, Hemizellulosen und Lignin umfasst Torf auch durch chemische und biologische Prozesse gebildete Umwandlungs- und Abbauprodukte wie Humusstoffe, Fulvosäuren und Bitumen. Ferner umfasst Torf anorganische Bestandteile (z.B. Mineralstoffe), welche u.a. von den Pflanzen selbst stammen.

Torf wird bereits seit vielen Jahrhunderten für verschiedenste Zwecke eingesetzt. Aufgrund seines im Wesentlichen pflanzlichen Ursprungs eignet sich Torf, in getrocknetem Zustand, besonders gut als Brennstoff. Ferner wurde Torf auch als isolierendes Füll- und Dämmmaterial im Häuserbau verwendet. Seit jeher dient Torf auch als Einstreu in Viehställen. Wegen der hohen spezifischen Wärme und der antiseptischen Wirkung verwendet man Torf auch für Moorbäder und bei rheumatischen Erkrankungen. Heute wird Torf zumeist als Kultursubstrat im Gartenbau eingesetzt.

Torf, wie hierin verwendet, wird vorzugsweise aus Mooren, insbesondere aus Hoch- und/oder Niedermooren, mit bekannten Verfahren gewonnen. Daher umfasst der Begriff "Torf" auch den umgangssprachlich häufig verwendeten Begriff "Moor", der sich auf Torf bezieht, auch wenn damit - lt. wissenschaftlicher Definition - ein Lebensraum bezeichnet wird, in dem sich Torf ablagert. Der Begriff "Torf" umfasst auch "Moortränke", "Moortorf" oder sonstige Torf umfassende Produkte umfassen.

Es wurde nun gefunden, dass Torf (wie auch Moortränke) und/oder Torf-haltige bzw. Torf umfassende Produkte in der Lage sind, Phosphonat, insbesondere freies Phosphonat, in einer phosphonathaltigen Zusammensetzung zu binden und somit in der Zusammensetzung zu reduzieren bzw. zu entziehen.

Das "phosphonathaltige Ausgangsmaterial" kann pflanzlicher und/oder tierischer Herkunft, anorganischer Herkunft (z.B. Erdreich), Wasser, eine Suspension, eine Lösung oder ein Gemenge verschiedenster Materialien sein. Ein "phosphonathaltiges Ausgangsmaterial" umfasst auch Futtermittel, das einem Tier verfüttert wird, wobei die Menge des im Futtermittel vorhandenen Phosphonats entweder vor oder während der Verdauung durch den erfindungsgemäßen Torf reduziert wird.

Die Menge an Torf, welches zum phosphonathaltigen Ausgangsmaterial, vorzugsweise Futtermittel, zugegeben wird, kann je nach der Menge des zu bindenden Phosphonats variiert werden. Vorzugsweise wird Torf dem Ausgangsmaterial im Verhältnis 10:1 (10 Teile Torf:1 Teil Ausgangsmaterial) bis 1:100 (1 Teil Torf:100 Teile Ausgangsmaterial), vorzugsweise 9:1 bis 1:90, noch mehr bevorzugt 8:1 bis 1:80, noch mehr bevorzugt 7:1 bis 1:70, noch mehr bevorzugt 6:1 bis 1:60, noch mehr bevorzugt 5:1 bis 1:50, noch mehr bevorzugt 4:1 bis 1:40, noch mehr bevorzugt 3:1 bis 1:30, noch mehr bevorzugt 2:1 bis 1:20, noch mehr bevorzugt 1:10, zugegeben.

"Reduzieren" bzw. "Reduktion" in Bezug auf die Menge an Phosphonat in einer phosphonathaltigen Zusammensetzung, wie hierin verwendet, bedeutet, dass die absolute Menge an Phosphonat, vorzugsweise freiem Phosphonat, in der Zusammensetzung um mindestens 10%, vorzugsweise um mindestens 20%, noch mehr bevorzugt um mindestens 30%, noch mehr bevorzugt um mindestens 40%, noch mehr bevorzugt um mindestens 50%, noch mehr bevorzugt um mindestens 60%, noch mehr bevorzugt um mindestens 70%, noch mehr bevorzugt um mindestens 80%, noch mehr bevorzugt um mindestens 90%, noch mehr bevorzugt um mindestens 95%, noch mehr bevorzugt um mindestens 98%, noch mehr bevorzugt um mindestens 99%, insbesondere um 100%, im Vergleich zum Ausgangsmaterial (d.h. der nicht mit Torf behandelten Zusammensetzung) verringert wird.

Der Begriff "freies Phosphonat" bezieht sich auf jenes Phosphonat in einer Zusammensetzung, welches für weitere chemische oder biologische Prozesse bzw. Reaktionen zur Verfügung steht. "Freies Phosphonat" kann durch im Stand der Technik bekannte Verfahren bestimmt werden (z.B. Gauch R et al (Zeitschrift für Lebensmittel-Untersuchung und Forschung 188 (1989):36-38)). Hat das aus dem Ausgangsmaterial zu entfernende bzw. zu reduzierende Phosphonat Einfluss auf das Wachstum von Mikroorganismen, kann auch dieses dazu verwendet werden, um "freies Phosphonat" zu bestimmen (siehe z.B. Shehata AA et al. Chemosphere 104(2014):258-61).

Ein weiterer Aspekt der vorliegenden Erfindung betrifft ein Verfahren zur Reduktion oder Entfernung von Phosphonat in einem phosphonathaltigen Ausgangsmaterial. Bei einem derartigen Verfahren wird der erfindungsgemäße Torf mit einem phosphonathaltigen Ausgangsmaterial über einen bestimmten Zeitraum (z.B. mindestens 1 Minute, vorzugsweise mindestens 5 Minuten, noch mehr bevorzugt mindestens 10 Minuten, noch mehr bevorzugt mindestens 30 Minuten, noch mehr bevorzugt mindestens 60 Minuten, noch mehr bevorzugt mindestens 5 Stunden, noch mehr bevorzugt mindestens 24 Stunden) in Kontakt gebracht.

Gemäß einer bevorzugten Ausführungsform der vorliegenden Erfindung umfasst der Torf und/oder das Torf-haltige bzw. Torf umfassende Produkt 3% bis 60% Huminstoffe bezogen auf die Trockenmasse des Torfs und/oder des Torf-haltigen bzw. Torf umfassenden Produkts.

Huminstoffe sind hochmolekulare Stoffe des Torfs, welche eine uneinheitliche makromolekulare Struktur aufweisen. Die Huminstoffe bestehen aus Kernen, Brücken und reaktiven Seitengruppen. Als Kerne kommen häufig Benzol, Indol, Pyrrol, Naphthalin, Pyridin, Chinolin oder auch Furan vor. Als Brücken fungieren vornehmlich Sauerstoff, Stickstoff, Kohlenstoff, einfache Kohlenwasserstoffe oder sekundäre Carbonsäureamide. Bei den Seitengruppen sind im Wesentlichen Carboxy-, Carbonyl-, Methoxy-, Amino- und Hydroxy-Gruppen beteiligt.

Huminstoffe umfassen Humine, Fulvosäuren und Huminsäuren. Als Humine werden die wasser- und alkaliunlöslichen Stoffe der Huminstoffe bezeichnet. Fulvosäuren sind vor allem im sauren Milieu gut löslich und weisen eine deutlich geringere molare Masse auf als Huminsäuren. Huminsäuren sind im basischen Bereich löslich und weisen eine molare Masse zwischen 2000 und 300.000 Dalton auf.

Huminsäuren sind bekannt dafür, dass sie in der Lage sind Phosphonate wie Glyphosat zu binden. Jedoch zeigte sich überraschend, dass Torf im Verhältnis zu Produkten wie Leonardit (siehe z.B. WO 2014/040590), die einen hohen Huminsäuregehalt aufweisen, eine signifikant höhere Glyphosat-Bindungskapzität aufweist (siehe Beispiel 1).

Der erfindungsgemäß eingesetzte Torf umfasst vorzugsweise 4% bis 60%, 5% bis 60%, 6% bis 60%, 7% bis 60%, 8% bis 60%, 9% bis 60%, 10% bis 60%, 12% bis 60%, 15% bis 60%, 18% bis 60%, 20% bis 60%, 25% bis 60%, 3% bis 55%, 5% bis 55%, 6% bis 55%, 7% bis 55%, 8% bis 55%, 9% bis 55%, 10% bis 55%, 12% bis 55%, 15% bis 55%, 18% bis 55%, 20% bis 55%, 25% bis 55%, 3% bis 50%, 5% bis 50%, 6% bis 50%, 7% bis 50%, 8% bis 50%, 9% bis 50%, 10% bis 50%, 12% bis 50%, 15% bis 50%, 18% bis 50%, 20% bis 50%, 25% bis 50%, 3% bis 45%, 5% bis 45%, 6% bis 45%, 7% bis 45%, 8% bis 45%, 9% bis 45%, 10% bis 45%, 12% bis 45%, 15% bis 45%, 18% bis 45%, 20% bis 45%, 25% bis 45%, Huminstoffe.

Verfahren zur Bestimmung der Huminstoffe sind dem Fachmann hinreichend bekannt. Beispielsweise können die Huminstoffe in einem getrockneten Torf, wie in Barsch H., Billwitz K. und Scholz E. (1984; Labormethoden in der physischen Geographie. VEB Hermann Haack; z.B. Seiten 81 bis 85) beschrieben, bestimmt werden. Dabei werden zunächst aus der Bodenprobe die Huminstoffe mit einem Extraktionsmittel extrahiert. Mit Natronlauge (z.B. 1 mol/l) lässt sich die Huminstofffraktion der Humine aufgrund ihrer Unlöslichkeit in dieser Natronlauge von der Fulvosäuren- und Huminsäurenfraktion abtrennen, da die Huminsäuren und Fulvosäuren bei diesem Vorgang in Lösung bleiben. Die Huminsäuren und Fulvosäuren lassen sich dann durch Zugabe von 10%iger Schwefelsäure voneinander trennen. Dabei erfolgt im Gegensatz zu den Fulvosäuren die Fällung der Huminsäuren. Die Unterschiede in der Löslichkeit beruhen auf den unterschiedlichen chemischen Eigenschaften der Huminsäuren und Fulvosäuren (siehe oben). Über die zusätzliche Bestimmung des Gesamtkohlenstoffgehalts im Torf kann auf den Huminstoffgehalt und auf den Gehalt der Humine, Fulvosäuren und Huminsäuren rückgeschlossen werden.

Die "Trockenmasse des Torfs", wie hier verwendet, wird ermittelt, in dem der erfindungsgemäß verwendete Torf zur Gänze (d.h. bis zur Gewichtskonstanz) getrocknet wird. Um im Torf etwaig eingeschlossenes Wasser zu entfernen, wird dieser vor der Trocknung vorzugsweise mechanisch aufgeschlossen (z.B. mit einem Homogenisator).

Gemäß einer weiteren bevorzugten Ausführungsform der vorliegenden Erfindung umfasst der Torf und/oder das Torf-haltige bzw. Torf umfassende Produkt 2% bis 40% Huminsäuren bezogen auf die Trockenmasse des Torfs und/oder dss Torf-haltigen bzw. Torf umfassenden Produkts.

Besonders vorteilhaft ist die Verwendung von Torf und/oder Torf-haltigen bzw. Torf umfassenden Produkten, welche 3% bis 40% Huminsäure umfassen, um Phophonate, insbesondere freie Phosphonate wie Glyphosat, aus einer Zusammensetzung zu reduzieren.

Der Torf und/oder das Torf-haltige bzw. Torf umfassende Produkt umfasst vorzugsweise 3% bis 40%, 4% bis 40%, 5% bis 40%, 6% bis 40%, 7% bis 40%, 8% bis 40%, 9% bis 40%, 10% bis 40%, 12% bis 40%, 15% bis 40%, 18% bis 40%, 20% bis 40%, 25% bis 40%, 2% bis 35%, 3% bis 35%, 4% bis 35%, 5% bis 35%, 6% bis 35%, 7% bis 35%, 8% bis 35%, 9% bis 35%, 10% bis 35%, 12% bis 35%, 15% bis 35%, 18% bis 35%, 20% bis 35%, 25% bis 35%, 2% bis 30%, 3% bis 30%, 4% bis 30%, 5% bis 30%, 6% bis 30%, 7% bis 30%, 8% bis 30%, 9% bis 30%, 10% bis 30%, 12% bis 30%, 15% bis 30%, 18% bis 30%, 20% bis 30%, 25% bis 30%, Huminsäure. Die Menge im Torf vorhandener Huminsäure kann nach dem oben genannten Verfahren bestimmt werden.

Der Aschegehalt des Torfs gibt den Anteil anorganischer Bestandteile im Torf an. Anorganische Bestandteile sind zumeist Mineralstoffe, die z.B. in den Pflanzen natürlicherweise vorkommen. Zusätzliche anorganische Bestandteile im Torf können von mineralischen Anschwemmungen aus dem Erdreich stammen. Erfindungsgemäß ist es von besonderem Vorteil, wenn die Menge an mineralischen Bestandteilen so gering wie möglich ist, um das Binden freier Phosphonate, insbesondere von freiem Glyphosat, effizient zu ermöglichen. Daher weist der Torf und/oder das Torf-haltige bzw. Torf umfassende Produkt, gemäß einer besonders bevorzugten Ausführungsform der vorliegenden Erfindung, einen Aschegehalt von weniger als 10% bezogen auf die Trockenmasse des Torfs auf.

Der Aschegehalt im erfindungsgemäß verwendeten Torf beträgt vorzugsweise weniger als 9%, vorzugsweise weniger als 8%, vorzugsweise weniger als 7%, vorzugsweise weniger als 6%, vorzugsweise weniger als 5%, vorzugsweise weniger als 4%, vorzugsweise weniger als 3%, vorzugsweise weniger als 2%, vorzugsweise weniger als 1%, bezogen auf die Trockenmasse des Torfs.

Gemäß einer bevorzugten Ausführungsform der vorliegenden Erfindung weist der verwendete Torf und/oder das verwendete Torf-haltige bzw. Torf umfassende Produkt einen Wassergehalt von 3% bis 80% auf.

Der Torf, der erfindungsgemäß eingesetzt werden kann, kann unterschiedliche Mengen an Wasser umfassen. Vorzugsweise beträgt der Wassergehalt 3% bis 75%, 3% bis 70%, 3% bis 65%, 3% bis 60%, 3% bis 55%, 3% bis 50%, 3% bis 45%, 3% bis 40%, 3% bis 35%, 3% bis 30%, 3% bis 25%, 3% bis 20%, 5% bis 80%, 5% bis 75%, 5% bis 70%, 5% bis 65%, 5% bis 60%, 5% bis 55%, 5% bis 50%, 5% bis 45%, 5% bis 40%, 5% bis 35%, 5% bis 30%, 5% bis 25%, 5% bis 20%, 10% bis 80%, 10% bis 75%, 10% bis 70%, 10% bis 65%, 10% bis 60%, 10% bis 55%, 10% bis 50%, 10% bis 45%, 10% bis 40%, 10% bis 35%, 10% bis 30%, 10% bis 25%, 10% bis 20%, 15% bis 80%, 15% bis 75%, 15% bis 70%, 15% bis 65%, 15% bis 60%, 15% bis 55%, 15% bis 50%, 15% bis 45%, 15% bis 40%, 15% bis 35%, 15% bis 30%, 15% bis 25%, 15% bis 20%, am meisten bevorzugt 12% bis 15%.

Gemäß einer weiteren bevorzugten Ausführungsform der vorliegenden Erfindung beträgt das Mengenverhältnis von Kalzium zu Phosphor im Torf 4,5:1 bis 7,5:1 und/oder das Mengenverhältnis von Kalium zu Natrium 7:1 bis 7,5:1 beträgt.

Wird Torf dazu verwendet Phosphonate, insbesondere freies Glyphosat, in einer Zusammensetzung zu binden, welche beispielsweise als Futtermittel oder Futtermittelzusatz eingesetzt wird, ist es von Vorteil, dass die oben genannten Mengenverhältnisse eingehalten werden, um ein ausgewogenes Verhältnis an Mineralstoffen im Futtermittel herzustellen.

Es ist besonders bevorzugt, Torf und/oder das Torf-haltige bzw. Torf umfassende Produkt in einer Menge dem Futtermittel zuzusetzen, die ausreicht, um im Futtermittel vorhandene Phosphonate (z.B. Glyphosat) in einer signifikanten Menge zu binden bzw. zu neutralisieren. Vorzugsweise beträgt der Anteil des erfindungsgemäßen Torfs an der Gesamtmenge an Futtermittel eines Tieres 0,1 bis 20%, vorzugsweise 0,2 bis 10%, noch mehr bevorzugt 0,3 bis 5%, noch mehr bevorzugt 0,4 bis 2%. Der Torf und/oder das Torf-haltige bzw. Torf umfassende Produkt kann dabei dem Futtermittel beigemengt oder getrennt davon dem Tier verabreicht werden.

Um die Anzahl an Mikroorganismen im Torf - zum Beispiel bei einer Vermengung des Torfs mit Futtermittel - zu reduzieren, ohne sie jedoch zu stark zu verringern, um die vorteilhaften Wirkungen vieler Mikroorganismen, insbesondere von Hefe oder verdauungsfördernder Bakterien, wird der Torf vorzugsweise bei einer Temperatur von 50-90°C, vorzugsweise von 60-80°C, und vorzugsweise bei Normaldruck getrocknet. Selbstverständlich ist es auch möglich höhere Temperaturen von 90°C bis 140°C oder 90°C bis 110°C zur Trocknung des Torfs zu verwenden. Vorzugsweise wird der Torf für 1 bis 10 Stunden, bevorzugt für 1,5 bis 7 Stunden, noch mehr bevorzugt für 2 bis 5 Stunden oder für 2 bis 3 Stunden, bis zum Erreichen des gewünschten Wassergehalts getrocknet.

Gemäß einer bevorzugten Ausführungsform der vorliegenden Erfindung ist das zu reduzierende Phosphonat Glyphosat. Es hat sich herausgestellt, dass Torf in der Lage ist vor allem freies Glyphosat in einer Zusammensetzung zu binden, um dadurch dessen Verfügbarkeit für chemische oder biologische Prozesse zu reduzieren.

Phosphonate werden u.a. als Herbizide (wie z.B. das Glyphosat) Korrosioninhibitoren, als Peroxidstabilisatoren und in Waschmitteln eingesetzt. Phosphonate sind bekannt dafür, dass diese biologisch schlecht abbaubar sind, obwohl gewisse Phosphonate auch in der Natur vorkommen. Da Phosphonate toxische Eigenschaften aufweisen können, ist es ein Bestreben, freies, d.h. biologisch und/oder chemisch verfügbare Phosphonate, insbesondere Glyphosat, in phosphonathaltigen oder potentiell phosphonathaltigen Zusammensetzungen zu reduzieren. Da Phosphonate wie Gylphosat, beispielsweise, als Herbizid eingesetzt werden, besteht die Möglichkeit, dass diese Phosphonate - durch die Verwendung von mit Phosphonaten behandelten Pflanzen als Futtermittel - von die gefütterten Tieren aufgenommen werden. Daher ist es besonders bevorzugt Torf zur Reduktion der Menge freier Phosphonate, insbesondere freiem Glyphosat, in Futtermittel einzusetzen.

Der praktische Einsatz von Torf und/oder eines Torf-haltigen bzw. Torf umfassenden Produkts in Futtermitteln bzw. Futtermittelzusätzen der erfindungsgemäßen Art hat zudem überraschender Weise gezeigt, dass Durchfall und Gelenksbeschwerden bei Heim- und Nutztieren erfolgreich behandelt werden konnten, und sich bei Kühen eine verringerte Neigung zur Zystenbildung eingestellt hat. Des Weiteren konnte eine Steigerung der Milchaufnahme bei Kälbern, sowie eine Verringerung der Zellzahl in der Milch von Kühen beobachtet werden. Insgesamt erwies sich Futtermittel mit Torf als Zusatz bei Säugetieren, insbesondere bei Wiederkäuern, als sehr bekömmlich, sodass eine verbesserte Futteraufnahme beobachtet werden konnte.

Erfindungsgemäß kann der Torf und/oder das Torf-haltige bzw. Torf umfassende Produkt in jeglicher Form dem phoshonathaltigen Ausgangsmaterial beigegeben werden. Besonders bevorzugt ist die Verwendung von Torf in Tabletten- oder Pelletsform, insbesondere wenn es sich bei dem Ausgangsmaterial um Futtermittel handelt. Bei der Pressung von Torf zu Pellets ist es von Vorteil, wenn der Torf einen anfänglichen Wassergehalt von, beispielsweise, 20% bis 30%, vorzugsweise von 24% bis 27%, aufweist. Im Zuge der Erwärmung aufgrund des Pressvorganges wird der Wassergehalt des Torfs entsprechend reduziert. Bei der Herstellung der Pellets können Presshilfsmittel, wie z.B. Maisstärke, zugegeben werden, um die Stabilität der hergestellten Pellets und die Pelletierfähigkeit von Torf zu steigern.

Die chemische Analyse eines erfindungsgemäßen Torfs hat für zwei Proben etwa folgende Zusammensetzung ergeben:

### PROBE 1:

| **Nährstoffe (g/kg)** | | Frischmasse | Trockenmasse |
|---|---|---|---|
| Trockenmasse | TM | 869 | 1000 |
| Rohprotein | RP | 108 | 124 |
| Rohfett | RFE | 19 | 22 |
| Rohfaser | RFA | 151 | 174 |
| N-freie Extraktst. | NFE | 435 | 501 |
| Rohasche | RA | 156 | 180 |

| **Mengenelemente (g/kg)** | | | |
|---|---|---|---|
| Kalzium | Ca | 3,7 | 4,3 |
| Phosphor | P | 0,5 | 0,6 |
| Magnesium | Mg | 1,1 | 1,3 |
| Kalium | K | 0,8 | 0,9 |
| Natrium | Na | 0,11 | 0,13 |

| **Spurenelemente (mg/kg)** | | | |
|---|---|---|---|
| Eisen | Fe | 9899,0 | 11391,3 |
| Mangan | Mn | 157,0 | 180,7 |
| Zink | Zn | 19,0 | 21,9 |
| Kupfer | Cu | 8,0 | 9,2 |

Das Mengenverhältnis von Kalzium zu Phosphor ergibt sich bei dieser Probe zu 7.4:1, und jenes von Kalium zu Natrium zu 7,3:1. Der pH-Wert ergab sich zu 3,4.

### PROBE 2:

| **Nährstoffe (g/kg)** | | Frischmasse | Trockenmasse |
|---|---|---|---|
| Trockenmasse | TM | 844 | 1000 |
| Rohprotein | RP | 97 | 115 |
| Rohfett | RFE | 26 | 31 |
| Rohfaser | RFA | 203 | 241 |
| N-freie Extraktst. | NFE | 419 | 496 |
| Rohasche | RA | 99 | 117 |

| **Mengenelemente (g/kg)** | | | |
|---|---|---|---|
| Kalzium | Ca | 3,7 | 4,4 |
| Phosphor | P | 0,6 | 0,7 |
| Magnesium | Mg | 0,8 | 0,9 |
| Kalium | K | 1,0 | 1,2 |
| Natrium | Na | 0,14 | 0,17 |

| **Spurenelemente (mg/kg)** | | | |
|---|---|---|---|
| Eisen | Fe | 8008,0 | 9488,2 |
| Mangan | Mn | 132,0 | 156,4 |
| Zink | Zn | 17,0 | 20,1 |
| Kupfer | Cu | 15,0 | 17,8 |

Das Mengenverhältnis von Kalzium zu Phosphor ergibt sich bei dieser Probe zu 6,17:1, und jenes von Kalium zu Natrium zu 7,1:1. Der pH-Wert ergab sich zu 4,5.

Der erfindungsgemäße Torf wird vorzugsweise zur Reduktion der Menge an Phosphonaten, insbesondere freien Phosphonaten wie Glyphosat, im Magen-Darm-Trakt eines Tieres verwendet.

Die Eigenschaft des Torfs Phosphonate wie Glyphosat zu binden kann genutzt werden, um zumindest weitgehend zu verhindern, dass Phosphonate über den Magen- und/oder Darmtrakt eines Tieres resorbiert werden. Dadurch können beispielsweise Erkrankungen bei Tieren, die aufgrund von Phosphonaten entstehen können, verhindert werden. Zudem wird die Menge an Phosphonaten im Fleisch oder in der Milch des Tieres ebenfalls signifikant reduziert.

Um die Menge an Phosphonaten, die über das Futtermittel aufgenommen wird, zu reduzieren, kann Torf als Futtermittelzusatz verwendet werden. In diesem Fall kann Torf dem Futtermittel z.B. in Form von Pellets oder Granulat zugegeben werden. Alternativ dazu kann Torf auch getrennt vom Futtermittel den Tieren verabreicht werden.

Gemäß einer bevorzugten Ausführungsform der vorliegenden Erfindung wird der erfindungsgemäße Torf als Futtermittelzusatz zur Reduktion der Menge an Phosphonaten, insbesondere Phosphonate wie Glyphosat, im Futtermittel verwendet.

Ein weiterer Aspekt der vorliegenden Erfindung betrifft die Verwendung von Torf und/oder Torf-haltigen bzw. Torf umfassenden Produkten als Medikament, insbesondere als oral zu verabreichendes Medikament im Veterinärbereich.

Durch die hervorragenden Eigenschaften Phosphonate, insbesondere Glyphosat, zu binden kann Torf und/oder Torf-haltige bzw. Torf umfassende Produkte auch als Medikamente eingesetzt werden. Vor allem eignet sich Torf zur Prävention von Erkrankungen, die in Zusammenhang mit Phosphonaten stehen, da vom Körper aufgenommenen Phosphonate an Torf gebunden und ausgeschieden werden, bevor die Phosphonate im Körper resorbiert werden oder die Darmflora schädigen. Nebenbei stehen Phosphonate in Verdacht genotoxisch, kanzerogen, zytotoxisch und teratogen zu sein.

Ein noch weiterer Aspekt der vorliegenden Erfindung betrifft Torf und/oder Torf-haltige bzw. Torf umfassende Produkte zur Verwendung bei der Behandlung und/oder Prävention von Erkrankungen, die durch Phosphonate, insbesondere Glyphosat, hervorgerufen werden, wobei Torf und/oder Torf-haltige bzw. Torf umfassende Produkte vorzugsweise oral im veterinärmedizinischen Bereich verabreicht werden kann

Ein Aspekt der vorliegenden Erfindung betrifft ein Futtermittel oder Futtermittelzusatz umfassend Torf und/oder Torf-haltige bzw. Torf umfassende Produkte wie hierin definiert.

Die vorliegende Erfindung wird anhand der folgenden Beispiele näher illustriert, ohne jedoch auf diese beschränkt zu sein.

### BEISPIELE:

### Beispiel 1: Untersuchung des Glyphosat-Bindungsvermögens von Torf

Die Fähigkeit von Torf Glyphosat zu binden wurde mit Hilfe eines Glyphosat-empfindlichen *Enterococcus faecalis*-Stammes untersucht. Im Zuge dieser Untersuchungen wurde 1 ml einer *E. faecalis*-Suspension mit einer Mischung aus einer definierten Menge an Glyphosat und 1 mg Torf versetzt.

### Versuchsdurchführung

Die Prüfung von Torf mit einem Wassergehalt von ca. 12 bis 18% (DryPEAT der Fa. Weichselbaum) auf Glyphosatbindungsvermögen erfolgte mit einem Glyphosat-sensitiven *Enterococcus faecalis* Stamm. 1 mg Torf wurde mit jeweils 0, 300, 600, 1200, 2400 und 5000 µg Glyphosat (Monsanto, USA) versetzt und jeweils einer 1 ml *E. faecalis*-Suspension (10⁵ KbE ml⁻¹) zugegeben. Die Kontrolle wurde mit 1 ml einer Lösung enthaltend 0, 300, 600, 1200, 2400 und 5000 µg Glyphosat/ml ohne die Zugabe von Torf durchgeführt. Die Suspensionen wurden über Nacht bei 37° C inkubiert. Das Wachstum der Enterokokken in den jeweiligen Suspensionen wurde anschließend durch Ausstrich der Suspension auf CATC-Agar (Citrat-Azid-Tween-Carbonat-Agar) überprüft.

### Ergebnisse

Die Ergebnisse der Untersuchungen sind in folgender Tabelle A dargestellt.

**Tabelle A. Neutralisierung der antibakteriellen Wirkung von Glyphosat auf E. faecalis durch Torf.**

| Probe | 0 µg/ml Glyphosat | 300 µg/ml Glyphosat | 600 µg/ml Glyphosat | 1200 µg/ml Glyphosat | 2400 µg/ml Glyphosat | 5000 µg/ml Glyphosat |
|---|---|---|---|---|---|---|
| Torf | +++ | +++ | +++ | +++ | +++ | ++ |
| Kontrolle | +++ | - | - | - | - | - |

| | | | | | | |
|---|---|---|---|---|---|---|
| +++ hochgradiges Wachstum, ++ mittelgradiges Wachstum, - kein Wachstum | | | | | | |

### Diskussion

Mit Hilfe der durchgeführten Untersuchungen konnte für Torf ein hochgradiges Bindungsvermögen für den geprüften herbiziden Wirkstoff Glyphosat festgestellt werden. 1 mg des Torfs war in der Lage mindestens 5000 µg Glyphosat zu binden bzw. zu neutralisieren. Ab dieser Menge Glyphosat nahm die Hemmung des Wachstums von *E. faecalis* durch freies Glyphosat marginal zu.

Die Eigenschaft des Torfs relativ hohe Mengen an Glyphosat zu binden/neutralisieren ist in Anbetracht des Standes der Technik besonders überraschend. Huminsäuren, welche ebenfalls Bestandteile des Torfs sind, sind bekannt für ihre Fähigkeit Glyphosat zu binden. Jedoch zeigen Produkte, welche eine hohe Konzentrationen an Huminsäuren umfassen, eine geringere Bindungsfähigkeit. So wird beispielsweise in der WO 2014/040590 beschrieben, dass 1 mg Leonardit lediglich in der Lage ist 2400 µg Glyphosat zu binden.

### Beispiel 2: Fütterungsversuche

Glyphosat wird von Tieren über das pflanzliche Futter aufgenommen. Um den Einfluss von Torf auf die Aufnahme von Glyphosat aus dem Futter zu untersuchen, wurden Fütterungsversuche durchgeführt.

Ein Milchkuhbestand (120 Kühe) in Schleswig Holstein mit Problemen in der Milchleistung, Bewegungsstörungen und Fruchtbarkeit wurde über 3 Wochen mit 100 g Torf (DryPEAT der Fa. Weichselbaum, Österreich) pro Tier und Tag gefüttert, wobei der Torf gemeinsam mit 500 ml Sauerkrautsaft verfüttert wurde. Vor der Verabreichung des Torfs wurden die Tiere über 4 Wochen pro Tier und Tag mit 200 g Pflanzenkohle, welche durch pyrolytische Verkohlung rein pflanzlicher Ausgangsstoffe hergestellt wurde, und 500ml Sauerkrautsaft gefüttert.

Um den Einfluss der Verabreichung von Torf auf den von den Tieren aus dem Futter aufgenommenen Glyphosat zu untersuchen, wurde der Glyphosatgehalt im Urin der Kühe zu verschiedenen Zeitpunkten bestimmt (siehe Tabelle B). Vor Beginn der Verabreichung der Pflanzenkohle bzw. des Torfs, erhielten die Kühe bereits über mehrere Wochen ein Glyphosat-haltiges Futtermittel verabreicht. Der Glyphosatgehalt zum Zeitpunkt des Beginns der Verabreichung der Pflanzenkohle und des Sauerkrautsaftes wird als Ausgangs- und Vergleichswert herangezogen (Woche 0). Nach 4 Wochen Verfütterung desselben Futtermittels mit Pflanzenkohle und Sauerkrautsaft wurde der Glyphosatgehalt im Urin derselben Tiere nochmals gemessen (Woche 4). Ab Woche 4 wurde für drei Wochen dasselbe Futtermittel in Kombination mit Torf und Sauerkrautsaft verfüttert und anschließend der Glyphosatgehalt im Urin bestimmt (Woche 7).

**Tabelle B. Ergebnisse des Vergleichs der Applikation von Pflanzenkohle und Torf in Kombination mit Sauerkrautsaft an Milchkühe**

| | Woche 0** Mittelwert | Woche 4*** Mittelwert | Woche 7**** Mittelwert | Referenz |
|---|---|---|---|---|
| Glyphosat (Urin) ng/ml | **8, 1** | **4, 5** | **2,6** | ≤ 2* |

| | | | | |
|---|---|---|---|---|
| *entspricht Glyphosatgehalt von Kühen aus gentechnikfreier Region ** Ausgangswert *** 4 Wochen 200g Pflanzenkohle + 500ml Sauerkrautsaft **** 3 Wochen 100g Torf + 500ml Sauerkrautsaft | | | | |

Glyphosat im Urin wurde mit einem ELISA Testkit von Abraxis (USA) bestimmt, wobei Urin zuvor 1:20 mit destilliertem Wasser verdünnt wurde.

### Diskussion

Im Feldversuch ist die Reduktion der Glyphosatausscheidung über den Urin nach nur drei Wochen Torf-Applikation hervorzuheben, die nahe an den Wert aus gentechnikfreien Regionen heranreicht.

## Patentansprüche

1. Verwendung von Torf zur Reduktion der Menge von Phosphonaten in einem phosphonathaltigen Ausgangsmaterial.

2. Verwendung nach Anspruch 1, **dadurch gekennzeichnet, dass** das zu reduzierende Phosphonat Glyphosat ist.

3. Verwendung nach Anspruch 1 oder 2, **dadurch gekennzeichnet, dass** der Torf 3% bis 60% Huminstoffe bezogen auf die Trockenmasse des Torfs umfasst.

4. Verwendung nach einem der Ansprüche 1 bis 3, **dadurch gekennzeichnet, dass** der Torf 2% bis 40% Huminsäuren bezogen auf die Trockenmasse des Torfs umfasst.

5. Verwendung nach einem der Ansprüche 1 bis 4, **dadurch gekennzeichnet, dass** der Torf einen Aschegehalt von weniger als 10% bezogen auf die Trockenmasse des Torfs aufweist.

6. Verwendung nach einem der Ansprüche 1 bis 5, **dadurch gekennzeichnet, dass** der Torf einen Wassergehalt von 2% bis 80%, vorzugsweise von 3 bis 60%, noch mehr bevorzugt von 3 bis 50%, noch mehr bevorzugt von 3 bis 40%, noch mehr bevorzugt von 3 bis 30%, noch mehr bevorzugt von 3 bis 25%, aufweist.

7. Verwendung nach einem der Ansprüche 1 bis 6, **dadurch gekennzeichnet, dass** das Mengenverhältnis von Kalzium zu Phosphor im Torf 4,5:1 bis 7,5:1 beträgt.

8. Verwendung nach einem der Ansprüche 1 bis 7, **dadurch gekennzeichnet, dass** das Mengenverhältnis von Kalium zu Natrium im Torf 7:1 bis 7,5:1 beträgt.

9. Verwendung nach einem der Ansprüche 1 bis 8, **dadurch gekennzeichnet, dass** der Torf einem Trocknungsvorgang von 50-90°C unterzogen wird.

10. Verwendung nach einem der Ansprüche 1 bis 9, **dadurch gekennzeichnet, dass** das phosphonathaltige Ausgangsmaterial ein Futtermittel ist.

11. Verwendung von Torf nach einem der Ansprüche 1 bis 10 zur Reduktion der Menge Phosphonate, insbesondere Glyphosat, im Magen-Darm-Trakt eines Tieres.

12. Verwendung von Torf nach einem der Ansprüche 1 bis 11 als Futtermittelzusatz zur Reduktion der Menge Phosphonate, insbesondere Glyphosat, im Futtermittel.

13. Torf zur Verwendung als Medikament.

14. Torf zur Verwendung bei der Behandlung und/oder Prävention von Erkrankungen, die durch Phosphonate, insbesondere Glyphosat, hervorgerufen werden.

15. Futtermittel oder Futtermittelzusatz umfassend Torf wie in einem der Ansprüche 1 bis 9 definiert.
